(19) Europäisches Patentamt
European Patent Office
Office européen des brevets

(11) Numéro de publication: **0 419 348 A1**

# (12) DEMANDE DE BREVET EUROPEEN

(21) Numéro de dépôt: **90402576.4**

(22) Date de dépôt: **19.09.90**

(51) Int. Cl.5: **C07D 498/04**, //(C07D498/04, 263:00,221:00)

(30) Priorité: **20.09.89 FR 8912324**

(43) Date de publication de la demande:
**27.03.91 Bulletin 91/13**

(84) Etats contractants désignés:
**AT BE CH DE DK ES FR GB GR IT LI LU NL SE**

(71) Demandeur: **SCIENCE ET ORGANISATION**
**31 rue du Pont**
**F-92200 Neuilly sur Seine(FR)**

(72) Inventeur: **Guillaumet, Gérald**
**2 rue Auguste Renoir**
**F-45100 Orléans(FR)**
Inventeur: **Flouzat, Christine**
**14 rue des Tanneurs**
**F-45100 Orléans(FR)**

## (54) Nouveaux procédé de synthèse de dérivés d'oxazolopyridines.

(57) Procédé de synthèse de dérivés de formule (I) :

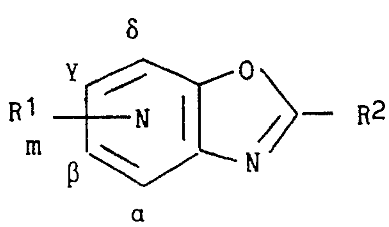

(I)

leurs sels de pyridinium et N - oxides dans laquelle :
- l'azote du cycle pyridine se situe en position $\alpha$, $\beta$, $\gamma$ ou $\delta$ de la jonction de cycle.
- $R^1$ représente un halogène, un alkyle substitué ou non, un alkoxy substitué ou non, un groupement nitro, amino substitué ou non, phényl, cyano.
- $0 \leq m \leq 2$.
- $R^2$ représente un groupement alkyle ou cycloakyle inférieur, un hétérocycle à 5 ou 6 chaînons contenant 1 ou 2 hétéroatomes substitués ou non, un groupement aryle

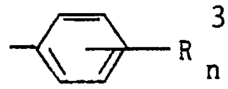

tel que :
- $R^3$ représente un halogène, un alkyle substitué ou non, un alkoxy substitué ou non, un groupement nitro, phényle, sulfonyle substitué ou non, cyano, thioalkyle, amino substitué ou non, sulfinyle substitué ou non, mercapto, hydroxy, ester,
- $0 \leq n \leq 5$
utilisant le triméthylsilylpolyphosphate (PPSE) comme agent de cyclisation et permettant l'obtention des dérivés de formule (I) avec des rendements quasi-quantitatifs.

EP 0 419 348 A1

# NOUVEAU PROCEDE DE SYNTHESE DE DERIVES D'OXAZOLOPYRIDINES

## NOUVEAU PROCEDE DE SYNTHESE DE DERIVES D'OXAZOLOPYRIDINES

La présente invention concerne un nouveau procédé de synthèse de dérivés d'oxazolopyridines. Plus spécifiquement, la présente invention concerne un nouveau procédé de synthèse de dérivés de formule générale (I) :

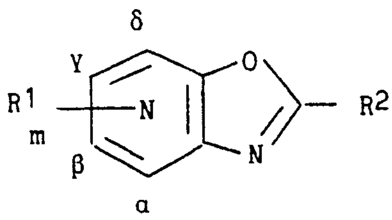

(I)

leurs sels de pyridinium et N - oxides dans laquelle :

- l'azote de cycle de pyridine se situe en position $\alpha$, $\beta$, $\gamma$ ou $\delta$ de la jonction de cycle.
- $R^1$ représente :
- un atome d'halogène,
- un groupement alkyle inférieur linéaire ou ramifié éventuellement substitué par un ou plusieurs atomes d'halogène tel que le groupement trifluorométhyle,
- un groupement alkoxy inférieur linéaire ou ramifié éventuellement substitué par un ou plusieurs atomes d'halogène tel que le groupement trifluorométhyle, - un groupement nitro,
- un groupement amino,
- un groupement benzoylamino,
- un groupement amino substitué par 1 ou 2 groupements alkyle inférieurs identiques ou différents,
- un groupement carbonylamino substitué par un alkoxy inférieur,
- un groupement phényl,
- un groupement cyano.
- $0 \leq m \leq 2$
- $R^2$ représente :

a. un groupement alkyle inférieur linéaire ou ramifié.

b. un groupement cycloalkyle inférieur linéaire ou ramifié.

c. un hétérocycle à 5 ou 6 chaînons contenant 1 ou 2 hétéroatomes tels que l'oxygène, l'azote ou le soufre substitué ou non par un groupement alkyle inférieur ou un groupement cyano.

d. un groupement aryle :

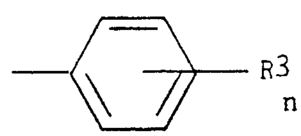

dans lequel :

- $0 \leq n \leq 5$
- $R^3$ représente :
- un halogène,
- un groupement alkyle inférieur linéaire ou ramifié éventuellement substitué par un ou plusieurs atomes d'halogène tel que le groupement trifluorométhyle,
- un groupement alkoxy linéaire ou ramifié éventuellement substitué par un ou plusieurs atomes d'halogène tel que le groupement trifluorométhyle,
- un groupement nitro,
- un groupement phényle,
- un groupement sulfonyle substitué par un alkyle inférieur,
- un groupement cyano,
- un groupement thioalkyle inférieur,
- un groupement carboxamide,
- un groupement amino substitué par un ou deux groupements alkyle inférieurs identiques ou différents,

- un groupement sulfinyle substitué par un alkyle inférieur,
- un groupement mercapto,
- un groupement alkanoyloxy inférieur,
- un groupement hydroxy,
- un groupement alkanoylamino inférieur,
- un groupement amino,
- un groupement benzoylamino substitué ou non par un halogène comme un chlore ou un brome ou un alkyle inférieur ou un alkoxy inférieur,

- 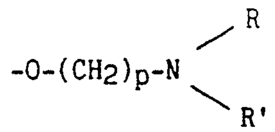

- 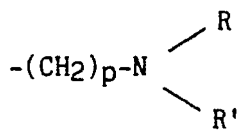

- 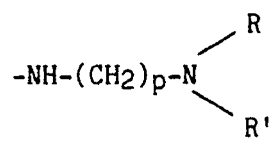

- 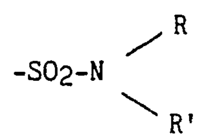

dans lesquels 1 ≦ p ≦ 3,
R, R′ identiques ou différents réprésentent un hydrogène ou un alkyle inférieur,
- un groupement ester $-CO_2R$ dans lequel R est un hydrogène ou un alkyle inférieur,
- $-(CH_2)_p-CO_2R$ avec p et R définis comme précédemment,
- $-(CH_2)_p-CN$ avec p défini comme précédemment,
- deux radicaux $R^3$ sur des carbones adjacents reliés entre eux pour former un méthylène dioxy.

Par radicaux alkyle, alkoxy, alkanoyloxy, alkanoylamino inférieur, on entend des groupements comportant 1 à 6 atomes de carbone.

Les dérivés obtenus sont également utiles en tant qu'intermédiaires de synthèse d'acyloxazolopyridines.

Un procédé de synthèse des dérivés de formule (I) a déjà été décrit dans le brevet britannique 1 421 619 et dans le Journal of Medicinal Chemistry, 1978, 21 , 11, 1158. Ces méthodes de synthèse permettent d'obtenir les dérivés de formule (I) avec des rendements globaux généralement compris entre 10 et 60 %.

La demanderesse a présentement découvert un procédé de synthèse des dérivés de formule (I) utilisant le triméthylsilylpolyphosphate (PPSE) comme agent en cyclisation au lieu d'acide polyphosphorique (PPA) fréquemment utilisé par exemple. Cette nouvelle méthode de synthèse, très simple à mettre en oeuvre, s'est révélée particulièrement avantageuse car, de façon surprenante, elle permet l'obtention des composés de formule (I) avec des rendements pratiquement quantitatifs donc nettement supérieurs à ceux obtenus jusqu'à présent.

Plus spécifiquement, le procédé selon la présente invention utilise comme produit de départ un dérivé de formule (II) :

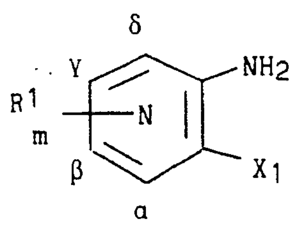

(II)

dans lequel :
- $X_1$ est un halogène,
- l'azote du cycle pyridine, se situe en position $\alpha$, $\beta$, $\gamma$ ou $\delta$ du carbone porteur de l'atome d'halogène,
- $R^1$ et m ont la même signification que dans la formule (I),

que l'on traite par un composé de formule (III) :

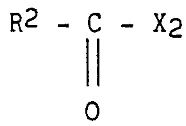

(III)

dans lequel :
- $X_2$ est un halogène,
- $R^2$ a la même signification que dans la formule (I),

pour conduire à un dérivé de formule (IV) :

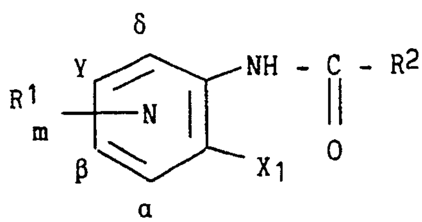

(IV)

dans lequel :
- $X_1$ est un halogène,
- l'azote du cycle pyridine se situe en position $\alpha$, $\beta$, $\gamma$ ou $\delta$ du carbone porteur de l'halogène,
- $R^1$, $R^2$ et m ont la même signification que dans la formule (I),

dont on porte à reflux une solution dans un solvant halogéné, pendant une période préférentiellement comprise entre 10 et 24 heures, en présence de triméthylsilylpolyphosphate (PPSE) obtenu de préférence extemporanément par reflux de pentoxyde de phosphore en présence d'hexaméthyldisyloxane, dans un solvant halogéné tel le dichloro - 1,2 benzène, sous atmosphère inerte, jusqu 'à obtention d'une solution limpide,

pour conduire aux dérivés de formule (I) que l'on purifie éventuellement par recristallisation ou chromatographie.

Les exemples cités ci-dessous illustrent l'invention mais ne la limitent en aucune façon.

**A. PREPRATION DE LA SOLUTION DE TRIMETHYLSILYLPOLYPHOSPHATE (PPSE)**

Cette solution est utilisée lors de la synthèse des dérivés de l'invention.

2,5 g pentoxyde de phosphore (7 mM) et 6,25 ml d'hexaméthyldisyloxane (33 mM) sont portés à reflux dans 12,5 ml de dichloro - 1,2 benzène pendant environ 5 minutes, sous atmosphère inerte ($N_2$, Ar), jusqu'à obtention d'une solution limpide.

Cette solution est utilisée telle quelle.

**EXEMPLE 1 : PHENYL - 2 OXAZOLO [5,4-b] PYRIDINE**

**STADE A : BENZOYLAMINO - 3 CHLORO - 2 PYRIDINE**

A 30 ml de pyridine, 3,9 g (28 mM) de chlorure de benzoyle sont ajoutés en maintenant la température à environ - 5 °C. A cette solution on ajoute, à l'aide d'une ampoule isobar, une solution contenant 3 g (23,3 mM) d'amino - 3 chloro - 2 pyridine dans 20 ml de pyridine. Le milieu réactionnel est laissé, sous agitation, pendant une nuit puis versé dans un mélange eau-glace. Le solide obtenu est alors filtré, lavé plusieurs fois à l'eau, puis recristallisé dans l'éthanol absolu.
Rendement : 92 %
Point de fusion : 89 °C

**STADE B : PHENYL - 2 OXAZOLO [5,4-b] PYRIDINE**

1,9 g (8 mM) de benzoylamino - 3 chloro - 2 pyridine, préparé au stade A sont ajoutés à la solution de PPSE puis portès à reflux pendant 15 heures. Le dichloro - 1,2 benzène est alors évaporé sous vide. Le résidu est additionné d'un mélange eau-glace. Le pH de la solution est ajusté à 7-8 avec une solution de bicarbonate de soude à 5 %. Le produit précipité est filtré puis recristallisé dans du cyclohexane.
Rendement : 96 %
Point de fusion : 101 °C

**EXEMPLE 2 : (FLUORO - 2 PHENYL) - 2 OXAZOLO [5,4-b] PYRIDINE**

**STADE A : (FLUORO - 2 BENZOYLAMINO) - 3 CHLORO - 2 PYRIDINE**

En procédant comme dans l'exemple 1, mais en remplaçant le chlorure de benzoyle par le chlorure de fluoro - 2 benzoyle, on obtient la (fluoro - 2 benzoylamino) - 3 chloro - 2 pyridine.
Rendement : 96 %
Solvant de recristallisation : isopropanol
Point de fusion : 122 °C

**STADE B : (FLUORO - 2 PHENYL) - 2 OXAZOLO [5,4-b] PYRIDINE**

En procédant comme dans l'exemple 1, mais en remplaçant la benzoylamino - 3 chloro - 2 pyridine par la (fluoro - 2 benzoylamino) - 3 chloro - 2 pyridine obtenue au stade A.
Rendement : 98 %
Solvant de recristallisation : éther/hexane
Point de fusion : 122 °C

**EXEMPLE 3 : (CHLORO - 4 PHENYL) - 2 OXAZOLO [5,4-b] PYRIDINE**

**STADE A : (CHLORO - 4 BENZOYLAMINO) - 3 CHLORO - 2 PYRIDINE**

En procédant comme dans l'exemple 1, mais en remplaçant le chlorure de benzoyle par le chlorure de chloro - 4 benzoyle, on obtient la (chloro - 4 benzoylamino) - 3 chloro - 2 pyridine.
Rendement : 95 %
Solvant de recristallisation : acétate d'éthyle/hexane
Point de fusion : 146 °C

**STADE B : (CHLORO - 4 PHENYL) - 2 OXAZOLO [5,4-b] PYRIDINE**

En procédant comme dans l'exemple 1, mais en remplaçant la benzoylamino - 3 chloro - 2 pyridine par la (chloro - 4 benzoylamino) - 3 chloro - 2 pyridine obtenue au stade A.

Rendement : 97%
Solvant de recristallisation : acétate d'éthyle/hexane Point de fusion : 152 °C

**EXEMPLE 4 : (CHLORO - 2 PHENYL) - 2 OXAZOLO [5,4-b] PYRIDINE**

**STADE A : (CHLORO - 2 BENZOYLAMINO) - 3 CHLORO - 2 PYRIDINE**

En procédant comme dans l'exemple 1, mais en remplaçant le chlorure de benzoyle par le chlorure de chloro - 2 benzoyle, on obtient la (chloro - 2 benzoylamino) - 3 chloro - 2 pyridine.
Rendement : 96 %
Solvant de recristallisation : éthanol absolu
Point de fusion : 109 °C

**STADE B : (CHLORO - 2 PHENYL) - 2 OXAZOLO [5,4-b] PYRIDINE**

En procédant comme dans l'exemple 1, mais en remplaçant la benzoylamino - 3 chloro - 2 pyridine par la (chloro - 2 benzoylamino) - 3 chloro - 2 pyridine obtenue au stade A. Rendement : 85 %
Solvant de recristallisation : acétate d'éthyle/hexane
Point de fusion : 116 °C

**EXEMPLE 5 : (METHOXY - 4 PHENYL) - 2 OXAZOLO [5,4-b] PYRIDINE**

**STADE A : (METHOXY - 4 BENZOYLAMINO) - 3 CHLORO - 2 PYRIDINE**

En procédant comme dans l'exemple 1, mais en remplaçant le chlorure de benzoyle par le chlorure de méthoxy - 4 benzoyle, on obtient la (méthoxy - 4 benzoylamino) - 3 chloro - 2 pyridine.
Rendement : 96 %
Solvant de recristallisation : isopropanol
Point de fusion : 125 °C

**STADE B : (METHOXY - 4 PHENYL) - 2 OXAZOLO [5,4-b] PYRIDINE**

En procédant comme dans l'exemple 1, mais en remplaçant la benzoylamino - 3 chloro - 2 pyridine par la (méthoxy - 4 benzoylamino) - 3 chloro - 2 pyridine obtenue au stade A.
Rendement 99 %
Solvant de recristallisation : hexane
Point de fusion : 144 °C

**EXEMPLE 6 : (NITRO - 2 PHENYL) - 2 OXAZOLO [5,4-b] PYRIDINE**

**STADE A : (NITRO - 2 BENZOYLAMINO) - 3 CHLORO - 2 PYRIDINE**

En procédant comme dans l'exemple 1, mais en remplaçant le chlorure de benzoyle par le chlorure de nitro - 2 benzoyle, on obtient la (nitro -2 benzoylamino) - 3 chloro - 2 pyridine.
Rendement : 96 %
Solvant de recristallisation : éthanol absolu
Point de fusion : 160 °C

**STADE B : (NITRO - 2 PHENYL) - 2 OXAZOLO [5,4-b] PYRIDINE**

En procédant comme dans l'exemple 1, mais en remplaçant la benzoylamino - 3 chloro - 2 pyridine par la (nitro - 2 benzoylamino) - 3 chloro - 2 pyridine obtenue au stade A.
Rendement : 77 %
Solvant de recristallisation : éther/cyclohexane
Point de fusion : 125 °C

**EXEMPLE 7 : (FURYL - 2) - 2 OXAZOLO [5,4-b] PYRIDINE**

**STADE A : (FURYL - 2 AMIDO) - 3 CHLORO - 2 PYRIDINE**

En procédant comme dans l'exemple 1, mais en remplaçant le chlorure de benzoyle par le chlorure de furoyle - 2, on obtient la (furyl - 2 amido) - 3 chloro - 2 pyridine.
Rendement : 75 %
Solvant de recristallisation : isopropanol
Point de fusion : 102 °C

**STADE B : (FURYL - 2) - 2 OXAZOLO [5,4-b] PYRIDINE**

En procédant comme dans l'exemple 1, mais en remplaçant la benzoylamino - 3 chloro - 2 pyridine par la (furyl - 2 amido) - 3 chloro -2 pyridine obtenue au stade A.
Rendement : 75 %
Solvant de recristallisation : cyclohexane
Point de fusion : 122 °C

**EXEMPLE 8 : (TRIFLUOROMETHYL - 4 PHENYL) - 2 OXAZOLO [5,4-b] PYRIDINE**

**STADE A : (TRIFLUOROMETHYL - 4 BENZOYLAMINO) - 3 CHLORO - 2 PYRIDINE**

En procédant comme dans l'exemple 1, mais en remplaçant le chlorure de benzoyle par le chlorure de trifluorométhyl - 4 benzoyle, on obtient la (trifluorométhyl - 4 benzoylamino - 3 chloro - 2 pyridine).

**STADE B : (TRIFLUOROMETHYL - 4 PHENYL) - 2 OXAZOLO [5,4-b] PYRIDINE**

En procédant comme dans l'exemple 1, mais en remplaçant la benzoylamino - 3 chloro - 2 pyridine par la (trifluorométhyl - 4 benzoylamino) - 3 chloro - 2 pyridine obtenue au stade A.

**EXEMPLE 9 : PHENYL - 2 OXAZOLO [4,5-b] PYRIDINE**

En procédant comme dans l'exemple 1, mais en remplaçant l'amino - 3 chloro - 2 pyridine par l'amino - 2 chloro - 3 pyridine.

**EXEMPLE 10 : (FLUORO - 2 PHENYL) - 2 OXAZOLO [4,5-b] PYRIDINE**

En procédant comme dans l'exemple 2, mais en remplaçant l'amino - 3 chloro - 2 pyridine par l'amino - 2 chloro - 3 pyridine.

**EXEMPLE 11 : (CHLORO - 4 PHENYL) - 2 OXAZOLO [4,5-b] PYRIDINE**

En procédant comme dans l'exemple 3, mais en remplaçant l'amino - 3 chloro - 2 pyridine par l'amino -

2 chloro - 3 pyridine.

**EXEMPLE 12 (CHLORO - 2 PHENYL) - 2 OXAZOLO [4,5-b] PYRIDINE**

En procédant comme dans l'exemple, 4 mais en remplaçant l'amino - 3 chloro - 2 pyridine par l'amino - 2 chloro - 3 pyridine.

**EXEMPLE 13 : (METHOXY - 4 PHENYL) - 2 OXAZOLO [4,5-b] PYRIDINE**

En procédant comme dans l'exemple 5, mais en remplaçant l'amino - 3 chloro - 2 pyridine par l'amino - 2 chloro - 3 pyridine.

**EXEMPLE 14 : (NITRO - 2 PHENYL) - 2 OXAZOLO [4,5-b] PYRIDINE**

En procédant comme dans l'exemple 6, mais en remplaçant l'amino - 3 chloro - 2 pyridine par l'amino - 2 chloro - 3 pyridine.

**EXEMPLE 15 : (FURYL - 2) - 2 OXAZOLO [4,5-b] PYRIDINE**

En procédant comme dans l'exemple 7, mais en remplaçant l'amino - 3 chloro - 2 pyridine par l'amino - 2 chloro - 3 pyridine.

**EXEMPLE 16 : (TRIFLUOROMETHYL - 4 PHENYL) - 2 OXAZOLO [4,5-b] PYRIDINE**

En procédant comme dans l'exemple 8 mais en remplaçant l'amino - 3 chloro - 2 pyridine par l'amino - 2 chloro - 3 pyridine.

**EXEMPLE 17 : PHENYL - 2 OXAZOLO [4,5-c] PYRIDINE**

En procédant comme dans l'exemple 1, mais en remplaçant l'amino - 3 chloro - 2 pyridine par l'amino - 3 chloro - 4 pyridine.

**EXEMPLE 18: (FLUORO - 2 PHENYL) - 2 OXAZOLO [4,5-c] PYRIDINE**

En prccédant comme dans l'exemple 2, mais en remplaçant l'amino - 3 chloro - 2 pyridine par l'amino - 3 chloro - 4 pyridine.

**EXEMPLE 19 : (CHLORO - 4 PHENYL) - 2 OXAZOLO [4,5-c] PYRIDINE**

En procédant comme dans l'exemple 3, mais en remplaçant l'amino - 3 chloro - 2 pyridine par l'amino - 3 chloro - 4 pyridine.

**EXEMPLE 20 : (CHLORO - 2 PHENYL) - 2 OXAZOLO [4,5-c] PYRIDINE**

En procédant comme dans l'exemple 4, mais en remplaçant l'amino - 3 chloro - 2 pyridine par l'amino - 3 chloro - 4 pyridine.

**EXEMPLE 21 : (METHOXY - 4 PHENYL) - 2 OXAZOLO [4,5-c] PYRIDINE**

En procédant comme dans l'exemple 5, mais en remplaçant l'amino - 3 chloro - 2 pyridine par l'amino - 3 chloro - 4 pyridine.

EXEMPLE 22 : (NITRO - 2 PHENYL) - 2 OXAZOLO [4,5-c] PYRIDINE

En procédant comme dans l'exemple 6, mais en remplaçant l'amino - 3 chloro - 2 pyridine par l'amino - 3 chloro - 4 pyridine.

**EXEMPLE 23 : (FURYL - 2) - 2 OXAZOLO [4,5-c] PYRIDINE**

En procédant comme dans l'exemple 7, mais en remplaçant l'amino - 3 chloro - 2 pyridine par l'amino - 3 chloro - 4 pyridine.

**EXEMPLE 24 : (TRIFLUOROMETHYL - 4 PHENYL) - 2 OXAZOLO [4,5-c] PYRIDINE**

En procédant comme dans l'exemple 8, mais en remplaçant l'amino - 3 chloro - 2 pyridine par l'amino - 3 chloro - 4 pyridine.

**EXEMPLE 25 : PHENYL - 2 OXAZOLO [5,4-c] PYRIDINE**

En procédant comme dans l'exemple 1, mais en remplaçant l'amino - 3 chloro - 2 pyridine par l'amino - 4 chloro - 3 pyridine.

**EXEMPLE 26 : (FLUORO - 2 PHENYL) - 2 OXAZOLO [5,4-c] PYRIDINE**

En procédant comme dans l'exemple 2, mais en remplaçant l'amino - 3 chloro - 2 pyridine par l'amino - 4 chloro - 3 pyridine.

**EXEMPLE 27 : (CHLORO - 4 PHENYL) - 2 OXAZOLO [5,4-c] PYRIDINE**

En procédant comme dans l'exemple 3, mais en remplaçant l'amino - 3 chloro - 2 pyridine par l'amino - 4 chloro - 3 pyridine.

**EXEMPLE 28 : (CHLORO - 2 PHENYL) - 2 OXAZOLO [5,4-c] PYRIDINE**

En procédant comme dans l'exemple 4, mais en remplaçant l'amino - 3 chloro - 2 pyridine par l'amino - 4 chloro - 3 pyridine.

**EXEMPLE 29 : (METHOXY - 4 PHENYL) - 2 OXAZOLO [5,4-c] PYRIDINE**

En procédant comme dans l'exemple 5, mais en remplaçant l'amino - 3 chloro - 2 pyridine par l'amino - 4 chloro - 3 pyridine.

**EXEMPLE 30 : (NITRO - 2 PHENYL) - 2 OXAZOLO [5,4-c] PYRIDINE**

En procédant comme dans l'exemple 6, mais en remplaçant l'amino - 3 chloro - 2 pyridine par l'amino - 4 chloro - 3 pyridine.

**EXEMPLE 31 : (FURYL - 2) - 2 OXAZOLO [5,4-c] PYRIDINE**

En procédant comme dans l'exemple 7, mais en remplaçant l'amino - 3 chloro - 2 pyridine par l'amino - 4 chloro - 3 pyridine.

**EXEMPLE 32 : (TRIFLUOROMETHYL - 4 PHENYL) - 2 OXAZOLO [5,4-c] PYRIDINE**

En procédant comme dans l'exemple 8, mais en remplaçant l'amino - 3 chloro - 2 pyridine par l'amino - 4 chloro - 3 pyridine.

**EXEMPLE 33 : CHLORO - 6 PHENYL - 2 OXAZOLO [4,5-b] PYRIDINE**

En procédant comme dans l'exemple 1, mais en remplaçant l'amino - 3 chloro - 2 pyridine par l'amino - 2 dichloro - 3,5 pyridine.

**EXEMPLE 34 : CHLORO - 6 (FLUORO - 2 PHENYL) - 2 OXAZOLO [4,5-b] PYRIDINE**

En procédant comme dans l'exemple 2, mais en remplaçant l'amino - 3 chloro - 2 pyridine par l'amino - 2 dichloro - 3,5 pyridine.

**EXEMPLE 35 : CHLORO - 6 (CHLORO - 4 PHENYL) - 2 OXAZOLO [4,5-b] PYRIDINE**

En procédant comme dans l'exemple 3, mais en remplaçant l'amino - 3 chloro - 2 pyridine par l'amino - 2 dichloro - 3,5 pyridine.

**EXEMPLE 36 : CHLORO - 6 (CHLORO - 2 PHENYL) - 2 OXAZOLO [4,5-b] PYRIDINE**

En procédant comme dans l'exemple 4, mais en remplaçant l'amino - 3 chloro - 2 pyridine par l'amino - 2 dichloro - 3,5 pyridine.

**EXEMPLE 37 : CHLORO - 6 (METHOXY - 4 PHENYL) - 2 OXAZOLO [4,5-bl PYRIDINE**

En procédant comme dans l'exemple 5, mais en remplaçant l'amino - 3 chloro - 2 pyridine par l'amino - 2 dichloro - 3,5 pyridine.

**EXEMPLE 38 : CHLORO - 6 (NITRO - 2 PHENYL) - 2 OXAZOLO [4,5-b] PYRIDINE**

En procédant comme dans l'exemple 6, mais en remplaçant l'amino - 3 chloro - 2 pyridine par l'amino - 2 dichloro - 3,5 pyridine.

**EXEMPLE 39 : CHLORO - 6 (FURYL - 2) - 2 OXAZOLO [4,5-b] PYRIDINE**

En procédant comme dans l'exemple 7, mais en remplaçant l'amino - 3 chloro - 2 pyridine par l'amino - 2 dichloro - 3,5 pyridine.

**EXEMPLE 40 : CHLORO - 6 (TRIFLUOROMETHYL - 4 PHENYL) - 2 OXAZOLO [4,5-b] PYRIDINE**

En procédant comme dans l'exemple 8, mais en remplaçant l'amino - 3 chloro - 2 pyridine par l'amino - 2 dichloro - 3,5 pyridine.

## Revendications

1/ Procédé de synthèse de composés de formule (I) :

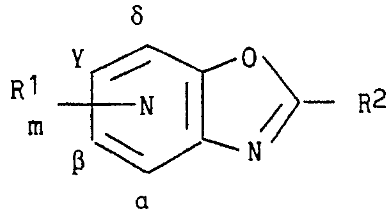

(I)

leurs sels de pyridinium et N - oxides dans laquelle :
- l'azote de cycle de pyridine se situe en position $\alpha$, $\beta$, $\gamma$ ou $\delta$ de la jonction de cycle.
- $R^1$ représente :
- un atome d'halogène,
- un groupement alkyle inférieur linéaire ou ramifié éventuellement substitué par un ou plusieurs atomes d'halogène tel que le groupement trifluorométhyle,
- un groupement alkoxy inférieur linéaire ou ramifié éventuellement substitué par un ou plusieurs atomes d'halogène tel que le groupement trifluorométhyle,
- un groupement nitro,
- un groupement amino,
- un groupement benzoylamino,
- un groupement amino substitué par 1 ou 2 groupements alkyle inférieurs identiques ou différents,
- un groupement carbonylamino substitué par un alkoxy inférieur,
- un groupement phényl,
- un groupement cyano.
- $0 \leqq m \leqq 2$
- $R^2$ représente :
a. un groupement alkyle inférieur linéaire ou ramifié.
b. un groupement cycloalkyle inférieur linéaire ou ramifié.
c. un hétérocycle à 5 ou 6 chaînons contenant 1 ou 2 hétéroatomes tels que l'oxygène, l'azote ou le soufre substitué ou non par un groupement alkyle inférieur ou un groupement cyano.
d. un groupement aryle :

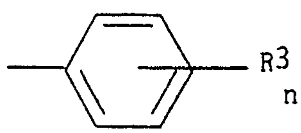

dans lequel :
- $0 \leqq n \leqq 5$
- $R^3$ représente :
- un halogène,
- un groupement alkyle inférieur linéaire ou ramifié éventuellement substitué par un ou plusieurs atomes d'halogène tel que le groupement trifluorométhyle,
- un groupement alkoxy linéaire ou ramifié éventuellement substitué par un ou plusieurs atomes d'halogène tel que le groupement trifluorométhyle,
- un groupement nitro,
- un groupement phényle,
- un groupement sulfonyle substitué par un alkyle inférieur,
- un groupement cyano,
- un groupement thioalkyle inférieur,
- un groupement carboxamide,
- un groupement amino substitué par 1 ou 2 groupements alkyle inférieurs identiques ou différents,
- un groupement sulfinyle substitué par un alkyle inférieur,
- un groupement mercapto,

- un groupement alkanoyloxy inférieur,
- un groupement hydroxy,
- un groupement alkanoylamino inférieur,
- un groupement amino,
- un groupement benzoylamino substitué ou non par un halogène comme un chlore ou un brome ou un alkyle inférieur ou un alkoxy inférieur,

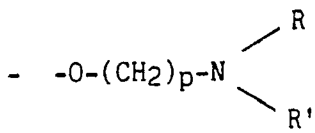

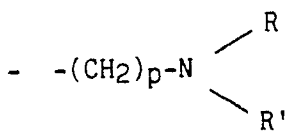

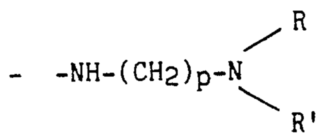

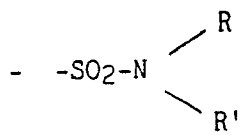

dans lesquels 1 ≦p ≦3,
R, R′ identiques ou différents réprésentent un hydrogène ou un alkyle inférieur,
- un groupement ester $-CO_2R$ dans lequel R est un hydrogène ou un alkyle inférieur,
- $-(CH_2)_p-CO_2R$ avec p et A définis comme précédemment,
- $-(CH_2)_p-CN$ avec p défini comme précédemment,
- deux radicaux $R^3$ sur des carbones adjacents reliés entre eux pour former un méthylène dioxy.
caractérisé en ce que l'on utilise comme produit de départ un dérivé de formule (II) :

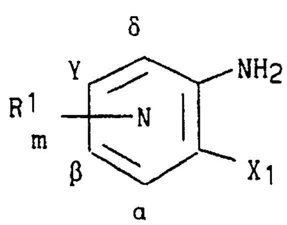

(II)

dans lequel :
- $X_1$ est un halogène,
- l'azote du cycle pyridine se situe en position $\alpha$, $\beta$, $\gamma$ ou $\delta$ du carbone porteur de l'halogène,
- $R^1$ et m ont la même signification que dans la formule (I),
que l'on traite par un composé de formule (III) :

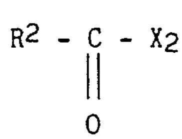

(III)

dans lequel :
- $X_2$ est un halogène,
- $R^2$ a la même signification que dans la formule (I),
pour conduire à un dérivé de formule (IV)

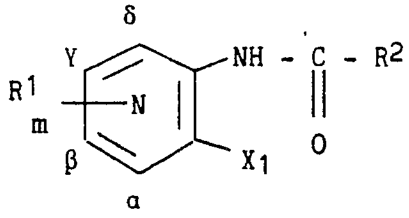

(IV)

dans lequel :
- $X_1$ est un halogène,
- l'azote du cycle pyridine se situe en postion $\alpha$, $\beta$, $\gamma$ ou $\delta$ du carbone porteur de l'halogène,
- $R^1$, $R^2$ et m ont la même signification que dans la formule (I),
que l'on traite par une solution de triméthylsilylpolyphosphate (PPSE) pour conduire à un dérivé de formule (I) que l'on purifie éventuellement par recristallisation ou chromatographie.

2/ Procédé de synthèse de dérivés de formule (I) selon la revendication 1, caractérisé en ce que la solution de PPSE que l'on met en réaction avec le dérivé de formule (IV) soit une solution de PPSE dans un solvant halogéné.

3/ Procédé de synthèse de dérivés de formule (I) selon l'une des revendications 1 ou 2, caractérisé en ce que la solution de PPSE que l'on met en réaction avec le dérivé de formule (IV) soit une solution de PPSE dans un solvant choisi parmi dichloro - 1,2 benzène ou dichloro - 1,2 éthane.

4/ Procédé de synthèse de dérivés de formule (I) selon l'une des revendications 1 ou 2, caractérisé en ce que le dérivé de formule (IV) est traité par une solution de PPSE à reflux.

5/ Procédé de synthèse de dérivés de formule (I) selon l'une des revendications 1 ou 2, caractérisé en ce que le dérivé de formule (IV) est traité par une solution de PPSE à reflux pendant une période comprise entre 10 et 24 heures.

6/ Procédé de synthèse de dérivés de formule (I) selon l'une des revendications 1 ou 2, caractérisé en ce que la solution de PPSE que l'on porte à réaction avec le dérivé de formule (IV) soit obtenue extemporanément par reflux de pentoxyde de phosphore en présence d'hexaméthyldisiloxane.

7/ Procédé de synthèse selon la revendication 1, permettant l'obtention de dérivés de formule (I) pour lesquels m = 0.

8/ Procédé de synthèse selon les revendication 1 et 7 permettant l'obtention de dérivés de formule (I) dans lesquels

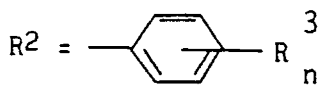

et tels que n = 0.

9/ Procédé de synthèse selon les revendications 1 et 7 permettant l'obtention de dérivés de formule (I) dans lesquels

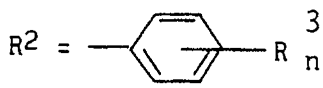

et tels que $R^3$ est un halogène et n =1.

10/ Procédé de synthèse selon l'une des revendications 1 et 7 permettant l'obtention de dérivés de formule (I) dans lesquels

$R^2$ =

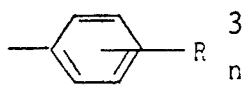

et tels que $R^3$ est un groupement alkyle inférieur linéaire ou ramifié éventuellement substitué par un ou plusieurs atomes d'halogène tel que le groupement trifluorométhyle et n = 1.

11/ Procédé de synthèse selon l'une des revendications 1 et 7 permettant l'obtention de dérivés de formule (I) dans lesquels

$R^2$ =

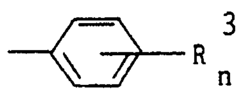

et tels que $R^3$ est un groupement alkoxy inférieur linéaire ou ramifié éventuellement substitué par un ou plusieurs atomes d'halogène tel que le groupement trifluorométhyle.

12/ Procédé de synthèse des dérivés de formule (I) selon l'une des revendications 1 et 7 permettant l'obtention d'aryl - 2 oxazolo [5,4-b] pyridine.

Revendications pour l'Etat contractant suivant: ES

1/ Procédé de synthèse de composés de formule (I) :

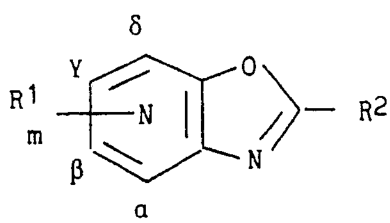

(I)

leurs sels de pyridinium et N - oxides dans laquelle :
- l'azote de cycle de pyridine se situe en position $\alpha$, $\beta$, $\gamma$ ou $\delta$ de la jonction de cycle.
- $R^1$ représente :
- un atome d'halogène,
- un groupement alkyle inférieur linéaire ou ramifié éventuellement substitué par un ou plusieurs atomes d'halogène tel que le groupement trifluorométhyle,
- un groupement alkoxy inférieur linéaire ou ramifié éventuellement substitué par un ou plusieurs atomes d'halogène tel que le groupement trifluorométhyle,
- un groupement nitro,
- un groupement amino,
- un groupement benzoylamino,
- un groupement amino substitué par 1 ou 2 groupements alkyle inférieurs identiques ou différents,
- un groupement carbonylamino substitué par un alkoxy inférieur,
- un groupement phényl,
- un groupement cyano.
- $0 \leqq m \leqq 2$ - $R^2$ représente :
a. un groupement alkyle inférieur linéaire ou ramifié.
b. un groupement cycloalkyle inférieur linéaire ou ramifié. c. un hétérocycle à 5 ou 6 chaînons contenant 1 ou 2 hétéroatomes tels que l'oxygène, l'azote ou le soufre substitué ou non par un groupement alkyle inférieur ou un groupement cyano.
d. un groupement aryle :

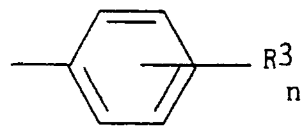

dans lequel :

- $0 \leq n \leq 5$
- $R^3$ représente :
- un halogène,
- un groupement alkyle inférieur linéaire ou ramifié éventuellement substitué par un ou plusieurs atomes d'halogène tel que le groupement trifluorométhyle,
- un groupement alkoxy linéaire ou ramifié éventuellement substitué par un ou plusieurs atomes d'halogène tel que le groupement trifluorométhyle,
- un groupement nitro,
- un groupement phényle,
- un groupement sulfonyle substitué par un alkyle inférieur,
- un groupement cyano,
- un groupement thioalkyle inférieur,
- un groupement carboxamide,
- un groupement amino substitué par 1 ou 2 groupements alkyle inférieurs identiques ou différents,
- un groupement sulfinyle substitué par un alkyle inférieur,
- un groupement mercapto,
- un groupement alkanoyloxy inférieur,
- un groupement hydroxy,

un groupement alkanoylamino inférieur,

- un groupement amino,
- un groupement benzoylamino substitué ou non par un halogène comme un chlore ou un brome ou un alkyle inférieur ou un alkoxy inférieur,

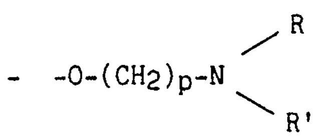

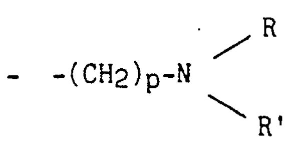

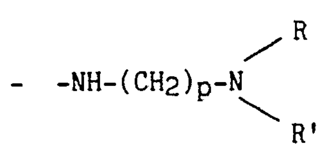

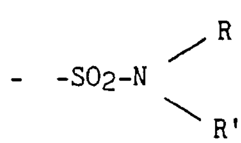

dans lesquels $1 \leq p \leq 3$,

R, R′ identiques ou différents réprésentent un hydrogène ou un alkyle inférieur,

- un groupement ester $-CO_2R$ dans lequel R est un hydrogène ou un alkyle inférieur,
- $-(CH_2)_p-CO_2R$ avec p et R définis comme précédemment,
- $-(CH_2)_p-CN$ avec p défini comme précédemment,

- deux radicaux $R^3$ sur des carbones adjacents reliés entre eux pour former un méthylène dioxy.
caractérisé en ce que l'on utilise comme produit de départ un dérivé de formule (II) :

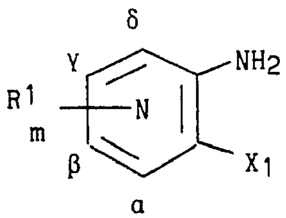

(II)

dans lequel :
- $X_1$ est un halogène,
- l'azote du cycle pyridine se situe en position $\alpha$, $\beta$, $\gamma$ ou $\delta$ du carbone porteur de l'halogène,
- $R^1$ et m ont la même signification que dans la formule (I),
que l'on traite par un composé de formule (III) :

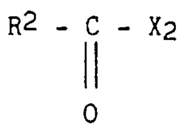

(III)

dans lequel :
- $X_2$ est un halogène,
- $R^2$ a la même signification que dans la formule (I),
pour conduire à un dérivé de formule (IV) :

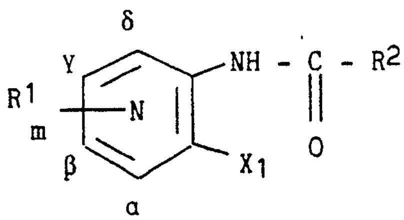

(IV)

dans lequel :
- $X_1$ est un halogène,
- l'azote du cycle pyridine se situe en postion $\alpha$, $\beta$, $\gamma$ ou $\delta$ du carbone porteur de l'halogène,
- $R^1$, $R^2$ et m ont la même signification que dans la formule (I),
que l'on traite par une solution de triméthylsilylpolyphosphate (PPSE) pour conduire à un dérivé de formule (I) que l'on purifie éventuellement par recristallisation ou chromatographie.

2/ Procédé de synthèse de dérivés de formule (I) selon la revendication 1, caractérisé en ce que la solution de PPSE que l'on met en réaction avec le dérivé de formule (IV) soit une solution de PPSE dans un solvant halogéné.

3/ Procédé de synthèse de dérivés de formule (I) selon l'une des revendications 1 ou 2, caractérisé en ce que la solution de PPSE que l'on met en réaction avec le dérivé de formule (V) soit une solution de PPSE dans un solvant choisi parmi dichloro - 1,2 benzène ou dichloro - 1,2 éthane.

4/ Procédé de synthèse de dérivés de formule (I) selon l'une des revendications 1 ou 2, caractérisé en ce que le dérivé de formule (IV) est traité par une solution de PPSE à reflux.

5/ Procédé de synthèse de dérivés de formule (I) selon l'une des revendications 1 ou 2, caractérisé en ce que le dérivé de formule (IV) est traité par une solution de PPSE à reflux pendant une période comprise entre 10 et 24 heures.

6/ Procédé de synthèse de dérivés de formule (I) selon l'une des revendications 1 ou 2, caractérisé en ce que la solution de PPSE que l'on porte à réaction avec le dérivé de formule (IV) soit obtenue extemporanément par reflux de pentoxyde de phosphore en présence d'hexaméthyldisiloxane.

7/ Procédé de synthèse selon la revendication 1, permettant l'obtention de dérivés de formule (I) pour

lesquels m = 0.

8/ Procédé de synthèse selon les revendication 1 et 7 permettant l'obtention de dérivés de formule (I) dans lesquels

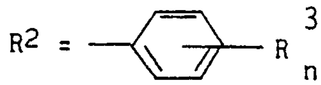

et tels que n = 0.

9/ Procédé de synthèse selon les revendications 1 et 7 permettant l'obtention de dérivés de formule (I) dans lesquels

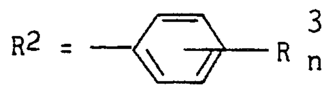

et tels que $R^3$ est un halogène et n = 1.

10/ Procédé de synthèse selon l'une des revendications 1 et 7 permettant l'obtention de dérivés de formule (I) dans lesquels

$R^2$ =

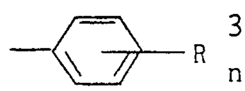

et tels que $R^3$ est un groupement alkyle inférieur linéaire ou ramifié éventuellement substitué par un ou plusieurs atomes d'halogène tel que le groupement trifluorométhyle et n = 1.

11/ Procédé de synthèse selon l'une des revendications 1 et 7 permettant l'obtention de dérivés de formule (I) dans lesquels

$R^2$ =

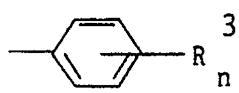

et tels que $R^3$ est un groupement alkoxy inférieur linéaire ou ramifié éventuellement substitué par un ou plusieurs atomes d'halogène tel que le groupement trifluorométhyle.

12/ Procédé de synthèse des dérivés de formule (I) selon l'une des revendications 1 et 7 permettant l'obtention d'aryl - 2 oxazolo [5,4-b] pyridine.

Pour L'Etat contractant suivant: GR

1/ Procédé de synthèse de composés de formule (I) :

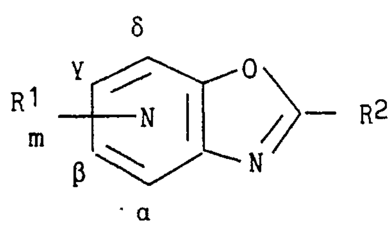

(I)

leurs sels de pyridinium et N - oxides dans laquelle :

- l'azote de cycle de pyridine se situe en position α, β, γ ou δ de la jonction de cycle.

- $R^1$ représente :
- un atome d'halogène,
- un groupement alkyle inférieur linéaire ou ramifié éventuellement substitué par un ou plusieurs atomes d'halogène tel que le groupement trifluorométhyle,
- un groupement alkoxy inférieur linéaire ou ramifié éventuellement substitué par un ou plusieurs atomes d'halogène tel que le groupement trifluorométhyle,
- un groupement nitro,
- un groupement amino,
- un groupement benzoylamino,
- un groupement amino substitué par 1 ou 2 groupements alkyle inférieurs identiques ou différents,
- un groupement carbonylamino substitué par un alkoxy inférieur,
- un groupement phényl,
- un groupement cyano.
- $0 \leq m \leq 2$
- $R^2$ représente :

a. un groupement alkyle inférieur linéaire ou ramifié.

b. un groupement cycloalkyle inférieur linéaire ou ramifié.

c. un hétérocycle à 5 ou 6 chaînons contenant 1 ou 2 hétéroatomes tels que l'oxygène, l'azote ou le soufre substitué ou non par un groupement alkyle inférieur ou un groupement cyano.

d. un groupement aryle :

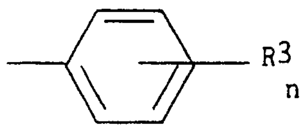

dans lequel :
- $0 \leq n \leq 5$
- $R^3$ représente :
- un halogène,
- un groupement alkyle inférieur linéaire ou ramifié éventuellement substitué par un ou plusieurs atomes d'halogène tel que le groupement trifluorométhyle,
- un groupement alkoxy linéaire ou ramifié éventuellement substitué par un ou plusieurs atomes d'halogène tel que le groupement trifluorométhyle,
- un groupement nitro,
- un groupement phényle,
- un groupement sulfonyle substitué par un alkyle inférieur,
- un groupement cyano,
- un groupement thioalkyle inférieur,
- un groupement carboxamide,
- un groupement amino substitué par 1 ou 2 groupements alkyle inférieurs identiques ou différents,
- un groupement sulfinyle substitué par un alkyle inférieur,
- un groupement mercapto,
- un groupement alkanoyloxy inférieur,
- un groupement hydroxy,
- un groupement alkanoylamino inférieur,
- un groupement amino,
- un groupement benzoylamino substitué ou non par un halogène comme un chlore ou un brome ou un alkyle inférieur ou un alkoxy inférieur,

- 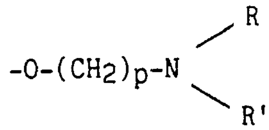

- 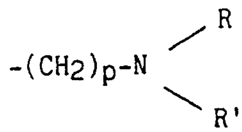

- 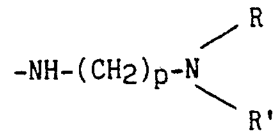

- 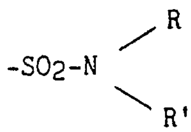

dans lesquels 1 ≦p ≦3,
R, R′ identiques ou différents réprésentent un hydrogène ou un alkyle inférieur,
- un groupement ester $-CO_2R$ dans lequel R est un hydrogène ou un alkyle inférieur,
- $-(CH_2)_p-CO_2R$ avec p et R définis comme précédemment,
- $-(CH_2)_p-CN$ avec p défini comme précédemment,
- deux radicaux $R^3$ sur des carbones adjacents reliés entre eux pour former un méthylène dioxy.
caractérisé en ce que l'on utilise comme produit de départ un dérivé de formule (II) :

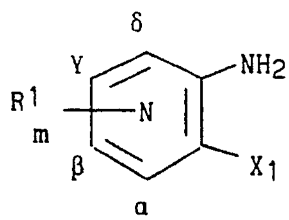

(II)

dans lequel :
- $X_1$ est un halogène,
- l'azote du cycle pyridine se situe en position $\alpha$, $\beta$, $\gamma$ ou $\delta$ du carbone porteur de l'halogène,
- $R^1$ et m ont la même signification que dans la formule (I),
que l'on traite par un composé de formule (III) :

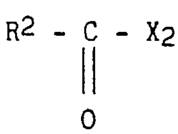

(III)

dans lequel :
- $X_2$ est un halogène,
- $R^2$ a la même signification que dans la formule (I), pour conduire à un dérivé de formule (IV) :

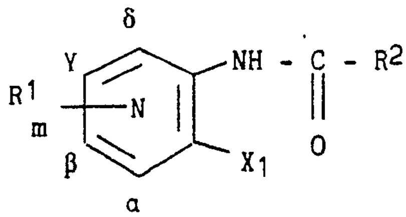

(IV)

dans lequel :

- $X_1$ est un halogène,
- l'azote du cycle pyridine se situe en postion $\alpha$, $\beta$, $\gamma$ ou $\delta$ du carbone porteur de l'halogène,
- $R^1$, $R^2$ et m ont la même signification que dans la formule (I),

que l'on traite par une solution de triméthylsilylpolyphosphate (PPSE) pour conduire à un dérivé de formule (I) que l'on purifie éventuellement par recristallisation ou chromatographie.

2/ Procédé de synthèse de dérivés de formule (I) selon la revendication 1, caractérisé en ce que la solution de PPSE que l'on met en réaction avec le dérivé de formule (IV) soit une solution de PPSE dans un solvant halogéné.

3/ Procédé de synthèse de dérivés de formule (I) selon l'une des revendications 1 ou 2, caractérisé en ce que la solution de PPSE que l'on met en réaction avec le dérivé de formule (IV) soit une solution de PPSE dans un solvant choisi parmi dichloro - 1,2 benzène ou dichloro - 1,2 éthane.

4/ Procédé de synthèse de dérivés de formule (I) selon l'une des revendications 1 ou 2, caractérisé en ce que le dérivé de formule (IV) est traité par une solution de PPSE à reflux.

5/ Procédé de synthèse de dérivés de formule (I) selon l'une des revendications 1 ou 2, caractérisé en ce que le dérivé de formule (IV) est traité par une solution de PPSE à reflux pendant une période comprise entre 10 et 24 heures.

6/ Procédé de synthèse de dérivés de formule (I) selon l'une des revendications 1 ou 2, caractérisé en ce que la solution de PPSE que l'on porte à réaction avec le dérivé de formule (IV) soit obtenue extemporanément par reflux de pentoxyde de phosphore en présence d'hexaméthyldisiloxane.

7/ Procédé de synthèse selon la revendication 1, permettant l'obtention de dérivés de formule (I) pour lesquels m = 0.

8/ Procédé de synthèse selon les revendication 1 et 7 permettant l'obtention de dérivés de formule (I) dans lesquels

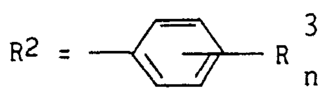

et tels que n = 0.

9/ Procédé de synthèse selon les revendications 1 et 7 permettant l'obtention de dérivés de formule (I) dans lesquels

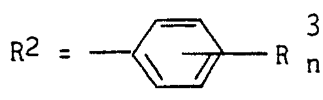

et tels que $R^3$ est un halogène et n = 1.

10/ Procédé de synthèse selon l'une des revendications 1 et 7 permettant l'obtention de dérivés de formule (I) dans lesquels

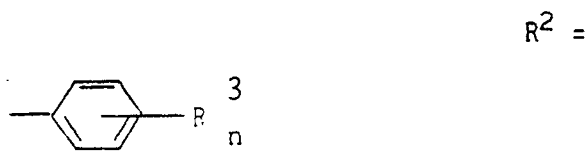

et tels que $R^3$ est un groupement alkyle inférieur linéaire ou ramifié éventuellement substitué par un ou plusieurs atomes d'halogène tel que le groupement trifluorométhyle et n = 1.

11/ Procédé de synthèse selon l'une des revendications 1 et 7 permettant l'obtention de dérivés de formule (I) dans lesquels

$R^2$ =

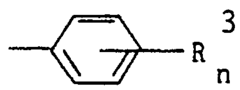

et tels que $R^3$ est un groupement alkoxy inférieur linéaire ou ramifié éventuellement substitué par un ou plusieurs atomes d'halogène tel que le groupement trifluorométhyle.

12/ Procédé de synthèse des dérivés de formule (I) selon l'une des revendications 1 et 7 permettant l'obtention d'aryl - 2 oxazolo [5,4-b] pyridine.

Office européen des brevets

# RAPPORT DE RECHERCHE EUROPEENNE

Numéro de la demande

**EP 90 40 2576**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | BULLETIN DE LA SOCIETE CHIMIQUE DE FRANCE, nos. 3-4, 1984, pages 11-142 - 11-144, Paris, FR; J.M. AIZPURUA et al.: "Reagents and synthetic methods. 27: improved synthesis of 2-substituted benzoxazoles induced by trimethylsilyl polyphosphate (PPSE)"<br>* Page 143, tableau 2, colonne 2, lignes 1-5; page 143, colonne 1, lignes 1-8 *<br>- - - | 1 | C 07 D 498/04 //<br>(C 07 D 498/04<br>C 07 D 263:00<br>C 07 D 221:00 ) |
| D,Y | FR-A-2 187 356 (MERCK)<br>* Revendication 1<br>* & GB-A-1 421 619<br>- - - - - | 1 | |
| | | | **DOMAINES TECHNIQUES RECHERCHES (Int. Cl.5)** |
| | | | C 07 D 498/00 |

Le présent rapport de recherche a été établi pour toutes les revendications

| Lieu de la recherche | Date d'achèvement de la recherche | Examinateur |
|---|---|---|
| La Haye | 17 décembre 90 | ALFARO I. |

CATEGORIE DES DOCUMENTS CITES

- X : particulièrement pertinent à lui seul
- Y : particulièrement pertinent en combinaison avec un autre document de la même catégorie
- A : arrière-plan technologique
- O : divulgation non-écrite
- P : document intercalaire
- T : théorie ou principe à la base de l'invention
- E : document de brevet antérieur, mais publié à la date de dépôt ou après cette date
- D : cité dans la demande
- L : cité pour d'autres raisons
- & : membre de la même famille, document correspondant